# EUROPÄISCHE PATENTSCHRIFT

(11) **EP 1 085 091 B1**
(45) Veröffentlichungstag und Bekanntmachung des Hinweises auf die Patenterteilung: **23.07.2003**
(21) Anmeldenummer: 00118053.8
(22) Anmeldetag: 23.08.2000
(51) Int. Cl.: C12N 15/31, C12N 15/52, C07K 14/34, C12N 1/21, C12P 13/08

(54) **Für das thrE-Gen kodierende Nukleotidsequenzen und Verfahren zur fermentativen Herstellung von L-Threonin mit coryneformen Bakterien**
Nucleotide sequences coding for the thrE-gene and process for the fermentative preparation of L-threonine using coryneform bacteria
Séquences de nucléotides codant pour le gène thrE et procédés de préparation de L-threonine utilisant des bactéries coryneformes

(30) Priorität: 01.09.1999 DE 19941478
(43) Veröffentlichungstag der Anmeldung: 21.03.2001
(73) Patentinhaber: Degussa AG, 40474 Düsseldorf (DE); Forschungszentrum Jülich GmbH, 52425 Jülich (DE)
(72) Erfinder: Thierbach, Georg, Dr., 33613 Bielefeld (DE); Ziegler, Petra, 52060 Aachen (DE); Eggeling, Lothar, Dr., 52428 Jülich (DE); Sahm, Hermann, Prof., 52428 Jülich (DE)

(56) Entgegenhaltungen:
- EP-A- 0 318 663
- EP-A- 0 593 792
- EP-A- 0 887 420
- WO-A-01/00805
- WO-A-01/00843
- WO-A-88/09819
- WO-A-93/09225
- DE-A- 19 831 609
- EIKMANNS B J ET AL: "MOLECULAR ASPECTS OF LYSINE, THREONINE, AND ISOLEUCINE BIOSYNTHESIS IN CORYNEBACTERIUM GLUTAMICUM" ANTONIE VAN LEEUWENHOEK,DORDRECHT,NL, Bd. 64, Nr. 2, 1993, Seiten 145-163, XP000918559
- MOLENAAR D ET AL: "BIOCHEMICAL AND GENETIC CHARACTERIZATION OF THE MEMBRANE-ASSOCIATEDMALATE DEHYDROGENASE (ACCEPTOR) FROM CORYNEBACTERIUM GLUTAMICUM" EUROPEAN JOURNAL OF BIOCHEMISTRY,BERLIN,DE, Bd. 254, 1998, Seiten 395-403, XP000941422 ISSN: 0014-2956

## Beschreibung

Gegenstand der Erfindung sind für das thrE-Gen kodierende Nukleotidsequenzen und Verfahren zur fermentativen Herstellung von L-Threonin unter Verwendung von coryneformen Bakterien, in denen das thrE-Gen verstärkt wird.

### Stand der Technik

L-Threonin findet in der Tierernährung, in der Humanmedizin und in der pharmazeutischen Industrie Anwendung.

Es ist bekannt, daß L-Threonin durch Fermentation von Stämmen coryneformer Bakterien insbesondere Corynebacterium glutamicum hergestellt werden kann. Wegen der großen Bedeutung wird ständig an der Verbesserung der Herstellverfahren gearbeitet. Verfahrensbesserungen können fermentationstechnische Maßnahmen wie z.B. Rührung und Versorgung mit Sauerstoff, oder die Zusammensetzung der Nährmedien wie z.B. die Zuckerkonzentration während der Fermentation, oder die Aufarbeitung zur Produktform durch z.B. Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Threonin-Analogon α-Amino-β-Hydroxyvaleriansäure (AHV) oder auxotroph für regulatorisch bedeutsame Aminosäuren sind und L-Threonin produzieren.

Seit einigen Jahren werden ebenfalls Methoden der rekombinanten DNA-Technik zur Stammverbesserung L-Threonin produzierender Stämme von Corynebacterium eingesetzt, indem man einzelne Threonin-Biosynthesegene amplifiziert und die Auswirkung auf die L-Threonin-Produktion untersucht.

### Aufgabe der Erfindung

Die Erfinder haben sich zur Aufgabe gestellt, neue Maßnahmen zur verbesserten fermentativen Herstellung von L-Threonin bereitzustellen.

### Beschreibung der Erfindung

L-Threonin findet in der Tierernährung, in der Humanmedizin und in der pharmazeutischen Industrie Anwendung. Es besteht daher ein allgemeines Interesse daran neue verbesserte Verfahren zur Herstellung von L-Threonin bereitzustellen.

Gegenstand der Erfindung ist eine in coryneformen Mikroorganismen replizierbare, rekombinante DNA mit der Herkunft Corynebacterium, die zumindest die Nukleotidsequenz enthält, die für das thrE-Gen, dargestellt in der SEQ-ID-No. 1 und SEQ-ID-No.3, codiert.

Gegenstand ist ebenfalls eine replizierbare DNA gemäß Anspruch 1 mit:
(i) den Nukleotidsequenzen, gezeigt in SEQ-ID-No. 1 oder SEQ-ID-No.3, die für das thrE-Gen codieren, oder
(ii) mindestens einer Sequenz, die den Sequenzen (i) innerhalb des Bereichs der Degeneration des genetischen Codes entspricht, oder
(iii) mindestens einer Sequenz, die mit den zu den Sequenzen (i) oder (ii) komplementären Sequenz hybridisiert, und/oder gegebenenfalls
(iv) funktionsneutralen Sinnmutationen in (i).

Ebenso sind coryneforme Mikroorganismen, insbesondere der Gattung Corynebacterium, transformiert durch die Einführung der genannten replizierbaren DNA Gegenstand der Erfindung.

Die Erfindung betrifft schließlich ein Verfahren zur fermentativen Herstellung von L-Threonin unter Verwendung von coryneformen Bakterien, die insbesondere bereits L-Threonin produzieren und in denen die für das thrE-Gen codierende(n) Nukleotidsequenz(en) verstärkt, insbesondere überexprimiert werden.

Der Begriff "Verstärkung" beschreibt in diesem Zusammenhang die Erhöhung der intrazellulären Aktivität eines oder mehrerer Enzyme in einem Mikroorganismus, die durch die entsprechende DNA kodiert werden, indem man beispielsweise die Kopienzahl des Gens bzw. der Gene erhöht, einen starken Promotor oder ein Gen verwendet, das für ein entsprechendes Enzym mit einer hohen Aktivität kodiert und gegebenenfalls diese Maßnahmen kombiniert.

Die Mikroorganismen, die Gegenstand der vorliegenden Erfindung sind, können L-Threonin aus Glucose, Saccharose, Lactose, Fructose, Maltose, Melasse, Stärke, Cellulose oder aus Glycerin und Ethanol herstellen. Es kann sich um Vertreter coryneformer Bakterien, insbesondere der Gattung Corynebacterium handeln. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind besonders die bekannten Wildtypstämme
Corynebacterium glutamicum ATCC13032
Corynebacterium acetoglutamicum ATCC15806
Corynebacterium acetoacidophilum ATCC13870
Corynebacterium melassecola ATCC17965
Corynebacterium thermoaminogenes FERM BP-1539
Brevibacterium flavum ATCC14067
Brevibacterium lactofermentum ATCC13869 und
Brevibacterium divaricatum ATCC14020
und daraus hergestellte L-Threonin produzierende Mutanten bzw. Stämme, wie beispielsweise
Corynebacterium glutamicum ATCC21649
Brevibacterium flavum BB69
Brevibacterium flavum DSM5399
Brevibacterium lactofermentum FERM-BP 269
Brevibacterium lactofermentum TBB-10

Den Erfindern gelang es, das thrE-Gen von Corynebacterium glutamicum zu isolieren. Zur Isolierung des thrE-Gens wird zunächst eine im thrE-Gen defekte Mutante von C. glutamicum hergestellt. Hierzu wird ein geeigneter Ausgangsstamm wie z. B. ATCC14752 oder ATCC13032 einem Mutagenese-Verfahren unterworfen.

Klassische Mutagenese-Verfahren sind die Behandlung mit Chemikalien wie z. B. N-Methyl-N-Nitro-N-Nitrosoguanidin oder UV-Bestrahlung. Derartige Verfahren zur Mutationsauslösung sind allgemein bekannt und können unter anderem bei Miller (A Short Course in Bacterial Genetics, A Laboratory Manual and Handbook for Escherichia coli and Related Bacteria (Cold Spring Harbor Laboratory Press, 1992)) oder im Handbuch "Manual of Methods for General Bacteriology" der American Society for Bacteriology (Washington D.C., USA, 1981) nachgelesen werden.

Ein anderes Mutagenese-Verfahren ist die Methode der Transposonmutagenese bei der die Eigenschaft eines Transposons ausgenutzt wird in DNA-Sequenzen zu"springen" und dadurch die Funktion des betreffenden Gens zu stören bzw. auszuschalten. Transposons coryneformer Bakterien sind in der Fachwelt bekannt. So wurden aus Corynebacterium xerosis Stamm M82B das Erythromycinresistenz-Transposon Tn5432 (Tauch et al., Plasmid (1995) 33: 168-179) und das Chloramphenicolresistenz-Transposon Tn5546 isoliert.

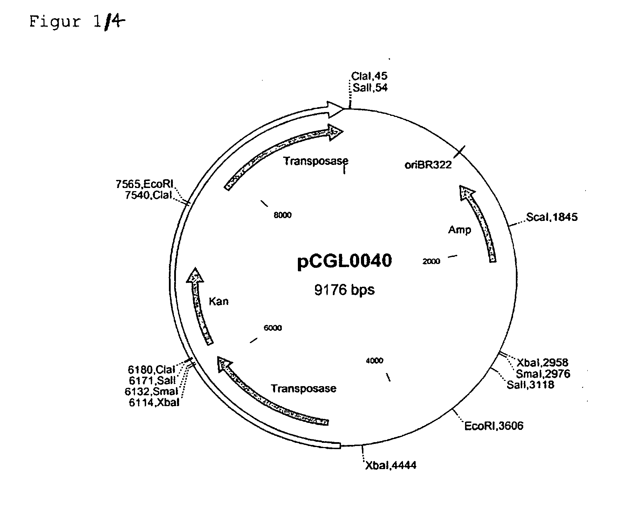

Ein anderes Transposon ist das Transposon Tn5531 das bei Ankri et al. (Journal of Bacteriology (1996) 178: 4412-4419) beschrieben wird und beispielhaft im Laufe der vorliegenden Erfindung eingesetzt wurde. Das Transposon Tn5531 enthält das aph3 Kanamycinresistenzgen und kann in Form des Plasmidvektors pCGL0040 verabreicht werden, der in Figur 1 dargestellt ist. Die Nukleotidsequenz des Transposons Tn5531 ist unter der Zugangsnummer (accession number) U53587 bei dem National Center for Biotechnology Information (NCBI, Bethesda, MD, USA) frei verfügbar.

Nach erfolgter Mutagenese vorzugsweise Transposon-Mutagenese wird eine im thrE-Gen defekte Mutante gesucht. Eine im thrE-Gen defekte Mutante wird daran erkannt, daß sie auf Minimalagar gutes Wachstum, aber auf Minimalagar, der mit Threonin haltigen Oligopeptiden wie z. B. dem Tripeptid Threonyl-threonyl-Threonin supplementiert wurde, schlechtes Wachstum zeigt.

Ein Beispiel für eine derartige Mutante ist der Stamm ATCC14752ΔilvAthrE::Tn5531.

Ein auf die beschriebene Weise hergestellter Stamm kann dann für die Isolierung und Klonierung des thrE-Gens verwendet werden.

Hierzu kann eine Genbank des interessierenden Bakteriums angelegt werden. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990) oder das Handbuch von Sambrook et al.: Molecular Cloning, A Laboratory Manual (Cold Spring Harbor Laboratory Press, 1989) genannt. Eine sehr bekannte Genbank ist die des E. coli K-12 Stammes W3110, die von Kohara et al. (Cell 50, 495 - 508 (1987)) in λ-Vektoren angelegt wurde. Bathe et al. (Molecular and General Genetics, 252:255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E.coli K-12 Stamm NM554 (Raleigh et al., 1988, Nucleic Acids Research 16:1563-1575) angelegt wurde. Für die vorliegende Erfindung eignen sich solche Vektoren, die in coryneformen Bakterien vorzugsweise Corynebacterium glutamicum replizieren. Derartige Vektoren sind aus dem Stand der Technik bekannt; als Beispiel sei der Plasmidvektor pZ1 genannt, der bei Menkel et al. (Applied and Environmental Microbiology (1989) 64: 549-554) beschrieben ist. Die auf die beschriebene Weise erhaltene Genbank wird anschließend mittels Transformation oder Elektroporation in den im thrE-Gen defekten Indikatorstamm überführt und solche Transformanten gesucht, die die Fähigkeit besitzen, in Gegenwart Threonin haltiger Oligopeptide auf Minimalagar zu wachsen. Das klonierte DNA-Fragment kann anschließend einer Sequenzanalyse unterzogen werden.

Bei Verwendung einer durch Tn5531-Mutagenese erzeugten Mutante eines coryneformen Bakteriums wie z. B. der Stamm ATCC14752ΔilvAthrE::Tn5531 kann das thrE::Tn5531-Allel direkt unter Ausnutzung des in ihm enthaltenen Kanamycinresistenzgens aph3 kloniert und isoliert werden. Hierzu verwendet man bekannte Kloniervektoren wie z. B. pUC18 (Norrander et al., Gene (1983) 26: 101-106 und Yanisch-Perron et al., Gene (1985) 33: 103-119). Als Klonierwirte eignen sich besonders solche E. coli-Stämme, die restriktions- und rekombinationsdefekt sind. Ein Beispiel hierfür ist der Stamm DH5αmcr, der von Grant et al. (Proceedings of the National Academy of Sciences USA, 87 (1990) 4645-4649) beschrieben wurde. Die Selektion auf Transformanten erfolgt in Gegenwart von Kanamycin. Die Plasmid-DNA der erhaltenen Transformanten wird anschließend sequenziert. Hierzu kann die von Sanger et al. (Proceedings of the National Academy of Sciences of the United States of America USA (1977) 74: 5463-5467) beschriebene Dideoxy-Kettenabbruchmethode verwendet werden. Hiernach erhält man die stromaufwärts und stromabwärts des Tn5531-Insertionsortes thrE-Gensequenzen. Die erhaltenen Nukleotidsequenzen werden dann mit kommerziell erhältlichen Sequenzanalyse-Programmen wie z. B.dem Programmpaket Lasergene (Biocomputing Software for Windows, DNASTAR, Madison, USA) oder dem Programmpaket HUSAR (Release 4.0, EMBL, Heidelberg, Deutschland) analysiert und zusammengefügt.

Auf diese Weise wurde die neue für das thrE-Gen kodierende DNA-Sequenz von C. glutamicum erhalten, die als SEQ ID NO 1 Bestandteil der vorliegenden Erfindung ist. Weiterhin wurde aus der vorliegenden DNA-Sequenz mit den oben beschriebenen Methoden die Aminosäuresequenz des entsprechenden Proteins abgeleitet. In SEQ ID NO 2 ist die sich ergebende Aminosäuresequenz des thrE-Genproduktes dargestellt.

Kodierende DNA-Sequenzen, die sich aus SEQ ID NO 1 durch die Degeneriertheit des genetischen Codes ergeben, sind ebenfalls Bestandteil der Erfindung. In gleicher Weise sind DNA-Sequenzen, die mit SEQ ID NO 1 oder Teilen von SEQ ID NO 1 hybridisieren, Bestandteil der Erfindung. In der Fachwelt sind weiterhin konservative Aminosäureaustausche wie z.B. Austausch von Glycin gegen Alanin oder von Asparaginsäure gegen Glutaminsäure in Proteinen als "Sinnmutationen" (sense mutations) bekannt, die zu keiner grundsätzlichen Veränderung der Aktivität des Proteins führen, d. h. funktionsneutral sind. Weiterhin ist bekannt, daß Änderungen am N- und/oder C-Terminus eines Proteins dessen Funktion nicht wesentlich beeinträchtigen oder sogar stabilisieren können. Angaben hierzu findet der Fachmann unter anderem bei Ben-Bassat et al. (Journal of Bacteriology 169:751-757 (1987)), bei O'Regan et al. (Gene 77:237-251 (1989)), bei Sahin-Toth et al. (Protein Sciences 3:240-247 (1994)), bei Hochuli et al. (Bio/Technology 6:1321-1325 (1988)) und in bekannten Lehrbüchern der Genetik und Molekularbiologie. Aminosäuresequenzen, die sich in entsprechender Weise aus SEQ ID NO 2 ergeben sind ebenfalls Bestandteil der Erfindung.

Unter Ausnutzung der in SEQ-ID-No. 1 dargestellten Nukleotidsequenz können geeignete Primer synthetisiert und diese dann dazu verwendet werden mit Hilfe der Polymerase-Kettenreaktion (PCR) thrE-Gene verschiedener coryneformer Bakterien und Stämme zu amplifizieren. Anleitungen hierzu findet der Fachmann unter anderem beispielsweise im Handbuch von Gait: Oligonukleotide synthesis: a practical approach (IRL Press, Oxford, UK, 1984) und bei Newton und Graham: PCR (Spektrum Akademischer Verlag, Heidelberg, Deutschland, 1994). Alternativ können die in SEQ-ID-No. 1 dargestellte Nukleotidsequenz oder Teile davon als Sonde zur Suche von thrE-Genen in Genbanken von insbesondere coryneformen Bakterien verwendet werden. Anleitungen hierzu findet der Fachmann unter anderem beispielsweise im Handbuch "The DIG System Users Guide for Filter Hybridization" der Firma Boehringer Mannheim GmbH (Mannheim, Deutschland, 1993) und bei Liebl et al. (International Journal of Systematic Bacteriology (1991) 41: 255-260) Die auf diese Weise amplifzierten thrE-Gen haltigen DNA-Fragmente werden anschließend kloniert und sequenziert.

Auf diese Weise wurde die in SEQ-ID-No. 3 dargestellte DNA-Sequenz des thrE-Gens des Stammes ATCC13032 erhalten, die ebenfalls Bestandteil der vorliegenden Erfindung ist. In SEQ-ID-No. 4 ist die sich ergebende Aminosäuresequenz dargestellt.

Gegenstand der Erfindung ist ebenfalls ein Verfahren zur Isolierung des thrE-Gens, dadurch gekennzeichnet, daß man als Indikatorstämme im thrE-Gen defekte Mutanten, vorzugsweise coryneformer Bakterien gewinnt, die auf einem ein Threonin-haltiges Oligopeptid enthaltenden Nährmedium nicht oder nur gering wachsen und
a) das thrE-Gen nach dem Anlegen einer Genbank identifiziert und isoliert, oder
b) im Fall der Transposon-Mutagenese auf das bevorzugt eine Antibiotikaresistenz enthaltende Transposon selektiert und so das thrE-Gen erhält.

Die Erfinder fanden heraus, daß coryneforme Bakterien nach Überexpression des thrE-Gens in verbesserter Weise L-Threonin produzieren.

Zur Erzielung einer Überexpression kann die Kopienzahl der entsprechenden Gene erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich die Expression im Verlaufe der fermentativen L-Threonin-Produktion zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in der Europäischen Patentschrift EPS 0 472 869, im US Patent 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in der Patentanmeldung WO 96/15246, bei Malumbres et al. (Gene 134, 15 - 24 (1993)), in der japanischen Offenlegungsschrift JP-A-10-229891, bei Jensen und Hammer (Biotechnology and Bioengineering 58, 191-195 (1998)), bei Makrides (Microbiological Reviews 60:512-538 (1996)) und in bekannten Lehrbüchern der Genetik und Molekularbiologie.

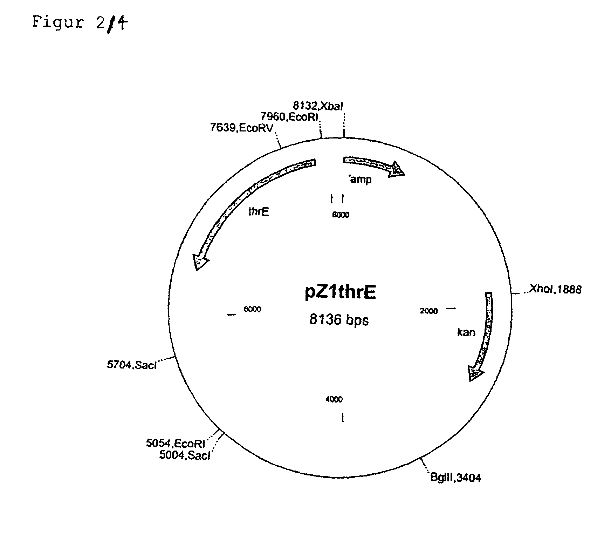

Ein Beispiel für ein Plasmid mit Hilfe dessen das thrE-Gen überexprimiert werden kann, ist pZ1thrE (Figur 2), das in dem Stamm DM368-2 pZ1thrE enthalten ist. Plasmid pZ1thrE ist ein auf Plasmid pZ1 beruhender C. glutamicum - E. coli Pendelvektor, der bei Menkel et al. (Applied and Environmental Microbiology (1989) 64: 549-554) beschrieben ist. Andere in C. glutamicum replizierbare Plasmidvektoren wie z.B. pEKEx1 (Eikmanns et al., Gene 102:93-98 (1991)) oder pZ8-1 (EP-B- 0 375 889) können in gleicher Weise verwendet werden.

Zusätzlich kann es für die Produktion von L-Threonin vorteilhaft sein neben dem neuen thrE-Gen ein oder mehrere Enzyme des bekannten Threonin-Biosyntheseweges oder Enzyme des anaplerotischen Stoffwechsels oder Enzyme des Zitronensäure-Zyklus zu überexprimieren. So kann beispielsweise:
- gleichzeitig das für die Homoserin-Dehydrogenase kodierende hom-Gen (Peoples et al., Molecular Microbiology 2, 63-72 (1988)) oder das für eine "feed back resistente" Homoserin-Dehydrogenase kodierende hom^{dr}-Allel (Archer et al., Gene 107, 53-59 (1991)) oder
- gleichzeitig das für die Pyruvat-Carboxylase kodierende pyc-Gen (DE-A-19 831 609) oder
- gleichzeitig das für die Malat:Chinon Oxidoreduktase kodierende mqo-Gen (Molenaar et al., European Journal of Biochemistry 254, 395 - 403 (1998))
überexprimiert werden.

Weiterhin kann es für die Produktion von L-Threonin vorteilhaft sein neben der Überexpression des thrE-Gens unerwünschte Nebenreaktionen wie z. B. die Threonin-Dehydrogenase auszuschalten (Nakayama: "Breeding of Amino Acid Producing Micro-organisms", in: Overproduction of Microbial Products, Krumphanzl, Sikyta, Vanek (eds.), Academic Press, London, UK, 1982 und Bell und Turner. Biochemical Journal 156, 449-458 (1976)).

Die erfindungsgemäß hergestellten Mikroorganismen können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Produktion von L-Threonin kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Stämme genügen. Beschreibungen von Kulturmedien verschiedenener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z.B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoff haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden. Als Phosphorquelle können Phosphorsäure, Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten werden Sauerstoff oder Sauerstoff haltige Gasmischungen wie z.B. Luft in die Kultur eingetragen. Die Temperatur der Kultur liegt normalerweise bei 20°C bis 45°C und vorzugsweise bei 25°C bis 40°C. Die Kultur wird solange fortgesetzt bis sich ein Maximum an L-Threonin gebildet hat. Dieses Ziel wird normalerweise innerhalb von 10 Stunden bis 160 Stunden erreicht.

Die Analyse von L-Threonin kann durch Anionenaustauschchromatographie mit anschließender Ninhydrin Derivatisierung erfolgen so wie bei Spackman et al. (Analytical Chemistry, 30, (1958), 1190)beschrieben, oder sie kann durch reversed phase HPLC erfolgen so wie bei Lindroth et al. (Analytical Chemistry (1979) 51: 1167-1174) beschrieben.

Folgende Mikroorganismen wurden bei der Deutschen Sammlung für Mikrorganismen und Zellkulturen (DSMZ, Braunschweig, Deutschland) gemäß Budapester Vertrag hinterlegt:
- Brevibacterium flavum Stamm DM368-2 pZlthrE als DSM 12840
- Escherichia coli Stamm GM2929pCGL0040 als DSM 12839

### Beispiele

Die vorliegende Erfindung wird im folgenden anhand von Ausführungsbeispielen näher erläutert.

Die Isolierung von Plasmid-DNA aus Escherichia coli sowie alle Techniken zur Restriktion, Klenow- und alkalische Phosphatasebehandlung wurden nach Sambrook et al. (Molecular cloning. A laboratory manual (1989) Cold Spring Harbour Laboratory Press) durchgeführt. Die Transformation von Escherichia coli wurde, wenn nicht anders beschrieben, nach Chung et al. (Proceedings of the National Academy of Sciences of the United States of America USA (1989) 86: 2172-2175) durchgeführt.

### Beispiel 1

### Klonierung und Sequenzierung des thrE-Gens von Corynebacterium glutamicum ATCC14752

### 1.Transposonmutagenese und Mutantenauswahl

Der Stamm Corynebacterium glutamicum ATCC14752ΔilvA wurde einer Mutagenese mit dem Transposon Tn5531 unterworfen, dessen Sequenz unter der Accession-Nummer U53587 in der Nukleotid-Datenbank des National Center for Biotechnology Information (Bethesda, USA) hinterlegt ist. Der Einbau einer Deletion in das ilvA-Gen von Corynebacterium glutamicum ATCC14752 wurde mit dem bei Schäfer et al. (Gene (1994) 145: 69-73) beschriebenen System zum Genaustausch durchgeführt. Dazu wurde der von Sahm et al. konstruierte Inaktivierungsvektor pK19mobsacBΔilvA (Applied and Environmental Microbiology (1999) 65: 1973-1979) zur Deletion verwendet. Zunächst wurde der Methylase-defekte Escherichia coli Stamm SCS110 (Jerpseth und Kretz, STRATEGIES in molecular biology 6, 22, (1993)) der Firma Stratagene (Heidelberg, Deutschland) mit 200 ng des Vektors pK19mobsacBΔilvA transformiert. Transformanten wurden anhand ihrer Kanamycinresistenz auf 50 µg/mL Kanamycin enthaltenden LB-Agarplatten identifiziert. Aus einer der Transformanten wurde das Plasmid pK19mobsacBΔilvA präpariert. Mittels Elektroporation (Haynes et al., FEMS Microbiology Letters (1989) 61: 329-334) wurden dieses Inaktivierungsplasmid dann in den Stamm Corynebacterium glutamicum ATCC14752 eingebracht. Klone, bei denen der Inaktivierungsvektor im Genom integriert vorlag, wurden anhand ihrer Kanamycinresistenz auf 15 µg/mL Kanamycin enthaltenden LBHIS-Agarplatten identifiziert (Liebl et al., FEMS Microbiology Letters (1989) 65: 299-304). Um auf die Excision des Vektors zu selektieren, wurden Kanamycinresistente Klone auf Saccharose-haltigem LBG-Medium (LB-Medium mit 15 g/L Agar , 2% Glukose und 10% Saccharose) ausplattiert. Dabei wurden Kolonien erhalten, welche den Vektor durch ein zweites Rekombinationsereignis wieder verloren haben (Jäger et al.; Journal of Bacteriology (1992) 174: 5462-5465). Durch Überimpfen auf Minimalmediumplatten (CGXII-Medium mit 15 g/L Agar (Keilhauer et al., Journal of Bacteriology (1993) 175: 5595-5603)) mit und ohne 300 mg/L L-Isoleucin, bzw. mit und ohne 50 µg/mL Kanamycin wurden sechs Klone isoliert, die durch Excision des Vektors Kanamycin sensitiv und Isoleucin auxotroph waren und bei denen nun das unvollständige ilvA-Gen (ΔilvA-Allel) im Genom vorlag. Einer dieser Klone wurde als Stamm ATCC14752ΔilvA bezeichnet und zur Transposonmutagenese eingesetzt.

Aus dem Methylase-defekten E. coli-Stamm GM2929pCGL0040 (E. coli GM2929: Palmer et al., Gene (1994) 143: 1-12) wurde das Plasmid pCGL0040 isoliert, welches das zusammengesetzte Transposon Tn5531 enthält (Ankri et al., Journal of Bacteriology (1996) 178: 4412-4419). Der Stamm Corynebacterium glutamicum ATCC14752ΔilvA wurde mittels Elektroporation (Haynes et al., FEMS Microbiology Letters (1989) 61: 329-334) mit dem Plasmid pCGL0040 transformiert. Klone, bei denen das Transposon Tn5531 ins Genom integriert war, wurden anhand ihrer Kanamycinresistenz auf 15 µg/mL Kanamycin enthaltenden LBHIS-Agarplatten identifiziert (Liebl et al., FEMS Microbiology Letters (1989) 65: 299-304). Auf diese Weise wurden 2000 Klone erhalten, welche auf verzögertes Wachstum in Anwesenheit von Threonyl-threonyl-threonin überprüft wurden. Dazu wurden alle Klone einzeln auf CGXII-Minimalmedium-Agarplatten mit und ohne 2 mM Threonyl-threonyl-threonin übertragen. Das Medium war identisch mit dem bei Keilhauer et al. beschriebenen Medium CGXII (Journal of Bacteriology (1993) 175: 5593-5603), enthielt aber zusätzlich 25 µg/mL Kanamycin, 300 mg/L L-Isoleucin und 15 g/L Agar. Die Zusammensetzung des von Keilhauer et al. beschriebenen Mediums ist in Tabelle 1 dargestellt.

**Tabelle 1**

| Zusammensetzung des Mediums CGXII | |
|---|---|
| Komponente | Konzentration |
| (NH₄)₂SO₄ | 20 g/L |
| Harnstoff | 5 g/L |
| KH₂PO₄ | 1 g/L |
| K₂HPO₄ | 1 g/L |
| MgSO₄ x 7 H₂O | 0,25 g/L |
| 3-Morpholinopropansulfonsäure | 42 g/L |
| CaCl₂ | 10 mg/L |
| FeSO₄ x 7 H₂O | 10 mg/L |
| MnSO₄ x H₂O | 10 mg/L |
| ZnSO₄ x 7H₂O | 1 mg/L |
| CuSO₄ | 0,2 mg/L |
| NiCl₂ x 6 H₂O | 0,02 mg/L |
| Biotin | 0,2 mg/L |
| Glukose | 40 g/L |
| Protokatechusäure | 30 mg/L |

Die Agarplatten wurden bei 30°C inkubiert und das Wachstum nach 12, 18 und 24 Stunden untersucht. Es wurde eine. Transposonmutante erhalten, die ohne Threonyl-threonyl-threonin vergleichbar mit dem Ausgangsstamm Corynebacterium glutamicum ATCC14752ΔilvA wuchs, in Anwesenheit von 2 mM Threonyl-threonyl-threonin aber verzögertes Wachstum zeigte. Diese wurde als ATCC14752ΔilvAthrE::Tn5531 bezeichnet.

### 2. Klonierung und Sequenzierung des Insertionsortes von Tn5531 in ATCC14752ΔilvAthrE::Tn5531

Um den stromaufwärts des Transposons Tn5531 gelegenen Insertionsort in der in Beispiel 1.1 beschriebenen Mutante zu klonieren, wurde zunächst die chromosomale DNA dieses Mutantenstammes wie bei Schwarzer et al. (Bio/Technology (1990) 9: 84-87) beschrieben isoliert und 400 ng davon mit der Restriktionsendonuklease EcoRI geschnitten. Der vollständige Restriktionsansatz wurde in den ebenfalls mit EcoRI linearisierten Vektor pUC18 (Norander et al., Gene (1983) 26: 101-106) der Firma Roche Diagnostics (Mannheim, Deutschland) ligiert. Mit dem gesamten Ligationsansatz wurde der E. coli Stamm DH5αmcr (Grant et al., Proceedings of the National Academy of Sciences of the United States of America USA (1990) 87: 4645-4649) mittels Elektroporation transformiert (Dower et al., Nucleic Acid Research (1988) 16: 6127-6145). Transformanten, bei denen auf dem Vektor pUC18 die Insertionsorte des Transposons Tn5531 kloniert vorlagen, wurden anhand ihrer Carbenicillin- und Kanamycinresistenz auf 50 µg/mL Carbenicillin und 25 µg/mL Kanamycin enthaltenden LB-Agarplatten identifiziert. Aus drei der Transformanten wurden die Plasmide präpariert und durch Restriktionsanalyse die Größen der klonierten Inserts bestimmt. Die Nukleotidsequenz des Insertionsortes auf einem der Plasmide mit einem ca. 5,7 kb großen Insert wurde nach der Dideoxy-Kettenabbruchmethode von Sanger et al. bestimmt (Proceedings of the National Academy of Sciences of the United States of America USA (1977) 74: 5463-5467). Hierzu wurden 2,2 kb des Inserts ausgehend von folgendem Oligonukleotid-Primer sequenziert: 5'-CGG GTC TAC ACC GCT AGC CCA GG-3'.

Zur Identifizierung des stromabwärts des Transposons gelegenen Insertionsortes wurde die chromosomale DNA der Mutante mit der Restriktionsendonuklease XbaI geschnitten und in den mit XbaI linearisierten Vektor pUC18 ligiert.
Die weitere Klonierung wurde wie oben beschrieben durchgeführt. Die Nukleotidsequenz des Insertionsortes auf einem der Plasmide mit einem ca. 8,5 kb großen Insert wurde nach der Dideoxy-Kettenabbruchmethode von Sanger et al. bestimmt (Proceedings of the National Academy of Sciences of the United States of America USA (1977) 74: 5463-5467). Hierzu wurden 0,65 kb des Inserts ausgehend von folgendem Oligonukleotid-Primer sequenziert: 5'-CGG TGC CTT ATC CAT TCA GG-3'.

Die erhaltenen Nukleotidsequenzen wurde mit dem Programmpaket Lasergene (Biocomputing Software for Windows, DNASTAR, Madison, USA) analysiert und zusammengefügt. Diese Nukleotidsequenz ist als SEQ ID NO 1 wiedergegeben. Die Analyse ergab die Identifizierung eines offenen Leserasters von 1467 bp Länge. Das entsprechende Gen wurde als thrE-Gen bezeichnet. Das dazugehörige Genprodukt umfasst 489 Aminosäuren und ist als SEQ ID NO 2 wiedergegeben.

### Beispiel 2

### Klonierung und Sequenzierung des Gens thrE aus Corynebacterium glutamicum ATCC13032

Das Gen thrE wurde in den E. coli Klonierungsvektor pUC18 (Norrander et al., Gene (1983) 26: 101-106, Roche Diagnostics, Mannheim, Deutschland) kloniert. Die Klonierung wurde in zwei Schritten durchgeführt. Zunächst wurde durch eine Polymerasekettenreaktion (PCR) das Gen aus Corynebacterium glutamicum ATCC13032 mittels folgender aus SEQ ID NO 1 abgeleiteter Oligonukleotid-Primer amplifiziert.
thrE-forward: 5'-CCC CTT TGA CCT GGT GTT ATT G-3' thrE-reverse:

5'-CGG CTG CGG TTT CCT CTT-3'

Die PCR-Reaktion wurde in 30 Zyklen in Gegenwart von 200 µM Deoxynukleotid-triphosphaten (dATP, dCTP, dGTP, dTTP), je 1 µM des entsprechenden Oligonukleotids, 100 ng chromosomaler DNA von Corynebacterium glutamicum ATCC13032, 1/10 Volumen 10-fach Reaktionspuffer und 2,6 Einheiten einer hitzestabilen Taq-/Pwo-DNA-Polymerase-Mischung (Expand High Fidelity PCR System der Firma Roche Diagnostics, Mannheim, Deutschland) in einem Thermocycler (PTC-100, MJ Research, Inc., Watertown, USA) unter folgenden Bedingungen durchgeführt: 94°C für 30 Sekunden, 58°C für 30 Sekunden und 72°C für 2 Minuten.

Das amplifizierte etwa 1,9 kb große Fragment wurde dann im folgenden mit Hilfe des SureClone Ligation Kit (Amersham Pharmacia Biotech, Uppsala, Schweden) nach Angaben des Herstellers in die SmaI-Schnittstelle des Vektors pUC18 ligiert. Mit dem gesamten Ligationsansatz wurde der E. coli Stamm DH5αmcr (Grant et al., Proceedings of the National Academy of Sciences of the United States of America USA (1990) 87: 4645-4649) transformiert. Transformanten wurden anhand ihrer Carbenicillinresistenz auf 50 µg/mL Carbenicillin enthaltenden LB-Agarplatten identifiziert.
Aus 8 der Transformanten wurden die Plasmide präpariert und durch Restriktionsanalyse auf das Vorhandensein des 1,9 kb PCR-Fragments als Insert überprüft. Das so entstandene rekombinante Plasmid wird im folgenden mit pUC18thrE bezeichnet.

Die Nukleotidsequenz des 1,9 kb PCR-Fragments in Plasmid pUC18thrE wurde nach der Dideoxy-Kettenabbruchmethode von Sanger et al. bestimmt (Proceedings of the National Academy of Sciences of the United States of America USA (1977) 74: 5463-5467). Hierzu wurde das vollständige Insert von. pUC18thrE mit Hilfe folgender Primer der Firma Roche Diagnostics (Mannheim, Deutschland) sequenziert.

Universalprimer:

5'-GTA AAA CGA CGG CCA GT-3'

Reverseprimer:

5'-GGA AAC AGC TAT GAC CAT G-3'

Die Nukleotidsequenz ist als SEQ ID NO 3 wiedergegeben. Die erhaltene Nukleotidsequenz wurde mit dem Programmpaket Lasergene (Biocomputing Software for Windows, DNASTAR, Madison, USA) analysiert. Die Analyse ergab die Identifizierung eines offenen Leserasters von 1467 bp Länge, das als thrE-Gen bezeichnet wurde. Dieses kodiert für ein Polypeptid von 489 Aminosäuren, welches als SEQ ID NO 4 wiedergegeben ist.

### Beispiel 3

Expression des Gens thrE in Corynebacterium glutamicum

Das unter Beispiel 2 beschriebene Gen thrE aus Corynebacterium glutamicum ATCC13032 wurde zur Expression in den Vektor pZ1 kloniert (Menkel et al., Applied and Environmental Microbiology (1989) 64: 549-554). Dazu wurde aus dem Plasmid pUC18thrE mit den Restriktionsenzymen SacI und XbaI ein 1881 bp großes, das Gen thrE enthaltendes DNA-Fragment ausgeschnitten. Die 5'- und 3'-Enden dieses Fragments wurden mit Klenow-Enzym behandelt. Das resultierende DNA-Fragment wurde in den zuvor mit ScaI linearisierten und dephosphorylierten Vektor pZ1 ligiert. Mit dem gesamten Ligationsansatz wurde der E. coli Stamm DH5αmcr (Grant et al., Proceedings of the National Academy of Sciences of the United States of America USA (1990) 87: 4645-4649) transformiert. Transformanten wurden anhand ihrer Kanamycinresistenz auf 50 µg/mL Kanamycin enthaltenden LB-Agarplatten identifiziert. Aus 2 Transformanten wurden die Plasmide präpariert und durch Restriktionsanalyse auf das Vorhandensein des 1881 bp ScaI/XbaI-Fragments als Insert überprüft. Das auf diese Weise entstandene rekombinante Plasmid wurde als pZ1thrE bezeichnet (Figur 2).

Mittels Elektroporation (Haynes et al., FEMS Microbiology Letters (1989) 61: 329-334) wurden die Plasmide pZ1 und pZ1thrE in den threoninbildenden Stamm Brevibacterium flavum DM368-2 eingebracht. Der Stamm DM368-2 ist in der EP-B-0 385 940 beschrieben und als DSM5399 hinterlegt. Transformanten wurden anhand ihrer Kanamycinresistenz auf 15 µg/mL Kanamycin enthaltenden LBHIS-Agarplatten identifiziert (Liebl et al., FEMS Microbiology Letters (1989) 65: 299-304). Auf diese Weise entstanden die Stämme Brevibacterium flavum DM368-2 pZ1 und DM368-2 pZ1thrE.

### Beispiel 5

### Herstellung von L-Threonin mit Brevibacterium flavum

Zur Untersuchung ihrer Threoninbildung wurden die Stämme B. flavum DM368-2 pZ1 und DM368-2 pZ1thrE in 100 mL Brain Heart Infusion-Medium mit 50 µg Kanamycin/mL (Difco Laboratories, Detroit, USA) für 14 Stunden bei 30°C vorkultiviert. Anschließend wurden die Zellen einmal mit 0,9%(w/v) Natriumchloridlösung gewaschen und mit dieser Suspension je 60 mL CgXII-Medium so angeimpft, daß die OD₆₀₀ (optische Dichte bei 600 nm) 0,5 betrug. Das Medium war identisch mit dem bei Keilhauer et al. beschriebenen Medium (Journal of Bacteriology (1993) 175: 5593-5603), enthielt aber zusätzlich 50 µg Kanamycin pro mL. Die Kultivierung beider Stämme wurde bei 30°C über einen Zeitraum von 72 Stunden durchgeführt. Nach 0, 24, 48 und 72 Stunden wurden jeweils Proben genommen und die Zellen kurz abzentrifugiert (5 Minuten bei 13000 Umdrehungen pro Minute mit einer Biofuge pico der Firma Heraeus, Osterode, Deutschland).

Die quantitative Bestimmung der extrazellulären Aminosäurekonzentrationen aus dem Kulturüberstand erfolgte mittels reversed phase HPLC (Lindroth et al., Analytical chemistry (1979) 51: 1167-1174). Es wurde ein HPLC-Gerät der Serie HP1100 (Hewlett-Packard, Waldbronn, Deutschland) mit angeschlossenem Fluoreszenzdetektor (G1321A) verwendet; die Systemsteuerung und die Auswertung der Daten erfolgte mit einer HP-Chem-Station (Hewlett-Packard). 1 µL der zu analysierenden Aminosäurelösung wurde in einer automatischen Vorsäulenderivatisierung mit 20 µL ortho-Phthalaldehyd/2-Mercaptoethanol-Fertigreagenz (Pierce Europe BV, Oud-Beijerland, Niederlande) gemischt. Die dabei entstehenden fluoreszierenden, thiosubstituierten Isoindole (Jones et al., Journal of Chromatography (1983) 266: 471-482) wurden über eine kombinierte Vorsäule (40x4 mm Hypersil ODS 5) und Hauptsäule (Hypersil ODS 5, beide Säulen von der Firma CS-Chromatographie Service GmbH, Langerwehe, Deutschland) mit einem Gradientenprogramm mit zunehmend unpolarer Phase (Methanol) aufgetrennt. Das polare Eluenz war Natriumacetat (0,1 Molar, pH 7,2); die Flußrate betrug 0,8 mL pro Minute. Die Fluoreszenzdetektion der derivatisierten Aminosäuren erfolgte bei einer Anregungswellenlänge von 230 nm und einer Emissionswellenlänge von 450 nm. Die Aminosäurekonzentrationen wurden über einen Vergleich mit einem externen Standard und Asparagin als zusätzlichem internen Standard berechnet.

Die Ergebnisse sind in Tabelle 2 aufgeführt.

**Tabelle 2**

| Stamm | L-Threonin (g/L) | | | |
|---|---|---|---|---|
| | 0 Std. | 24 Std. | 48 Std. | 72 Std. |
| DM368-2 pZ1 | 0 | 0,46 | 1,27 | 1,50 |
| DM368-2 pZ1thrE | 0 | 0,68 | 1,71 | 2,04 |

Folgende Figuren sind beigefügt:

### Beispiel 6

### Herstellung des Vektors pEC-T18mob2thrE

### 1. Konstruktion von pEC-T18mob2

Nach dem Stand der Technik wurde der E. coli - C. glutamicum Shuttle-Vektor pEC-T18mob2 konstruiert.

Der Vektor enthält die Replikationsregion rep des Plasmids pGA1 einschließlich des Replikationseffectors per (US-A-5,175,108; Nesvera et al., Journal of Bacteriology 179, 1525-1532 (1997)), das Tetracyclinresistenz vermittelnde tetA(Z)-Gen des Plasmids pAG1 (US-A- 5,158,891; Genbank-Eintrag beim National Center for Biotechnology Information (NCBI, Bethesda, MD, USA) mit der accession number AF121000), die Replikationsregion oriV des Plasmids pMB1 (Sutcliffe, Cold Spring Harbor Symposium on Quantitative Biology 43, 77-90 (1979)), das lacZα-Genfragment einschließlich des lac-Promotors und einer Mehrfachklonierschnittstelle (multiple cloning site, mcs) (Norrander et al. Gene 26, 101-106 (1983)) und die mob-Region des Plasmids RP4 (Simon et al.,(1983) Bio/Technology 1:784-791).

Der konstruierte Vektor wurde in den E. coli Stamm DH5α (Hanahan, In: DNA cloning. A practical approach. Vol. I. IRL-Press, Oxford, Washington DC, USA, 1985) transformiert. Die Selektion von Plasmid-tragenden Zellen erfolgte durch Ausplattieren des Transformationsansatzes auf LB Agar (Sambrook et al., Molecular cloning: a laboratory manual. 2^{nd} Ed. Cold Spring Harbor Laboratory Press, Cold Spring Harbor, N.Y., USA, 1989), der mit 5 mg/l Tetracyclin supplementiert worden war. Plasmid-DNA wurde aus einer Transformante mit Hilfe des QIAprep Spin Miniprep Kit der Firma Qiagen isoliert und durch Restriktion mit dem Restriktionsenzym EcoRI und HindIII anschließender Agarosegel-Elektrophorese (0,8%) überprüft.

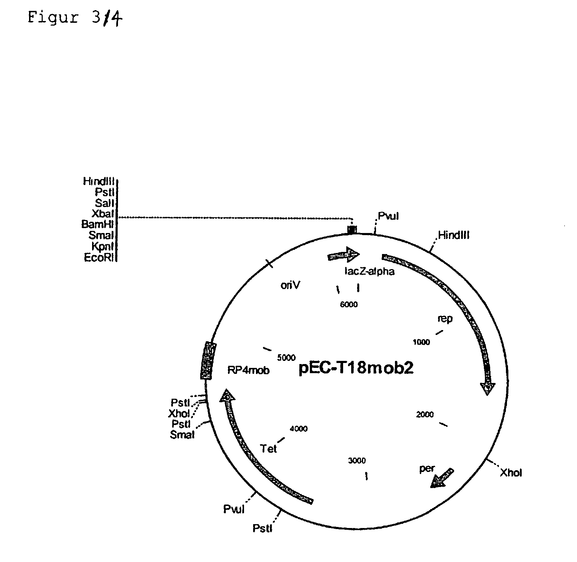

Das Plasmid wurde pEC-T18mob2 genannt und ist in Figur 3 dargestellt.

### 2. Konstruktion von pEC-T18mob2thrE

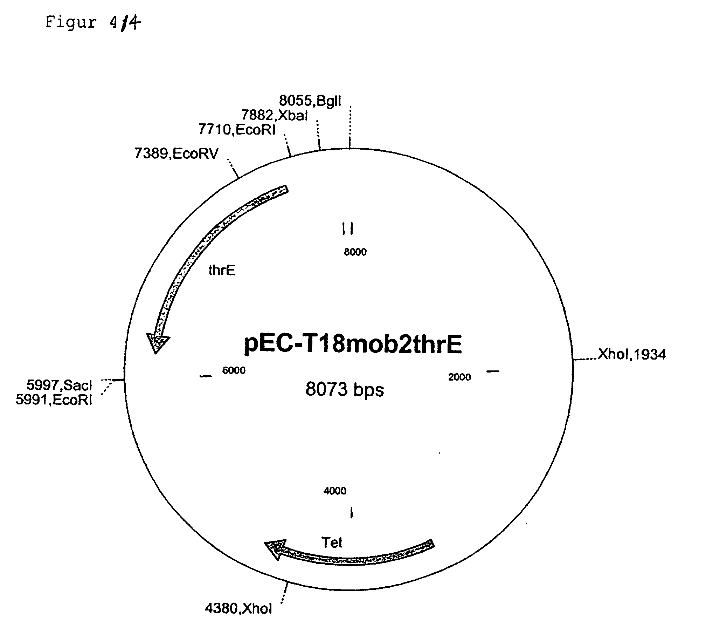

Das unter Beispiel 2 beschriebene Gen thrE aus Corynebacterium glutamicum ATCC13032 wurde zur Expression in den Vektor pEC-T18mob2 kloniert . Dazu wurde aus dem Plasmid pUC18thrE mit den Restriktionsenzymen SacI und XbaI ein 1881 bp großes, das Gen thrE enthaltendes DNA-Fragment ausgeschnitten. Das resultierende DNA-Fragment wurde in den zuvor mit SacI und XbaI linearisierten und dephosphorylierten Vektor pEC-T18mob2 ligiert. Mit dem gesamten Ligationsansatz wurde der E. coli Stamm DH5αmcr (Grant et al., Proceedings of the National Academy of Sciences of the United States of America USA (1990) 87: 4645-4649) transformiert. Transformanten wurden anhand ihrer Tetracyclinresistenz auf 5 µg/mL Tetracyclin enthaltenden LB-Agarplatten identifiziert. Aus 2 Transformanten wurden die Plasmide präpariert und durch Restriktionsanalyse auf das Vorhandensein des 1881 bp ScaI/XbaI-Fragments als Insert überprüft. Das auf diese Weise entstandene rekombinante Plasmid wurde als pEC-T18mob2thrE bezeichnet (Figur 4).

Mittels Elektroporation (Haynes et al., FEMS Microbiology Letters (1989) 61: 329-334) wurden die Plasmide pEC-T18mob2 und pEC-T18mob2thrE in den threoninbildenden Stamm Corynebacterium glutamicum MH20-22B-DR17 (Reinscheid et al., Applied and Environmental Microbiology (1994) 60: 126-132)eingebracht. Transformanten wurden anhand ihrer Tetracyclin- und Kanamycinresistenz auf 15 µg/mL Kanamycin und 5 µg/mL Tetracyclin enthaltenden LBHIS-Agarplatten identifiziert (Liebl et al., FEMS Microbiology Letters (1989) 65: 299-304). Auf diese Weise entstanden die Stämme Corynebacterium glutamicum MH20-22B-DR17/pEC-T18mob2·und MH20-22B-DR17/pEC-T18mob2thrE.

### Beispiel 7

### Herstellung von L-Threonin mit Corynebacterium glutamicum

Zur Untersuchung ihrer Threoninbildung wurden die Stämme C. glutamicum MH20-22B-DR17/pEC-T18mob2 und MH20-22B-DR17/pEC-T18mob2thrE in 100 mL Brain Heart Infusion-Medium mit 25 µg Kanamycin/mL und 5 µg Tetracyclin/mL (Difco Laboratories, Detroit, USA) für 14 Stunden bei 30°C vorkultiviert. Anschließend wurden die Zellen einmal mit 0,9%(w/v) Natriumchloridlösung gewaschen und mit dieser Suspension je 60 mL CgXII-Medium so angeimpft, daß die OD₆₀₀ (optische Dichte bei 600 nm) 0,5 betrug. Das Medium war identisch mit dem bei Keilhauer et al. beschriebenen Medium (Journal of Bacteriology (1993) 175: 5593-5603), enthielt aber zusätzlich 25 µg Kanamycin und 5 µg Tetracyclin pro mL. Die Kultivierung beider Stämme wurde bei 30°C über einen Zeitraum von 72 Stunden durchgeführt. Nach 0, 24, 48 und 72 Stunden wurden jeweils Proben genommen und die Zellen kurz abzentrifugiert (5 Minuten bei 13000 Umdrehungen pro Minute mit einer Biofuge pico der Firma Heraeus, Osterode, Deutschland).

Die quantitative Bestimmung der extrazellulären Aminosäurekonzentrationen aus dem Kulturüberstand erfolgte wie bereits in Beispiel 5 für Brevibacterium flavum beschrieben mittels reversed phase HPLC (Lindroth et al., Analytical chemistry (1979) 51: 1167-1174). Die Aminosäurekonzentrationen wurden über einen Vergleich mit einem externen Standard und Asparagin als zusätzlichem internen Standard berechnet.

Die Ergebnisse sind in Tabelle 3 aufgeführt.

**Tabelle 3**

| Stamm | L-Threonin (g/L) | | | |
|---|---|---|---|---|
| | 0 Std. | 24 Std. | 48 Std. | 72 Std. |
| MH20-22B-DR17/pEC-T18mob2 | 0 | 3,42 | 5,82 | 5,78 |
| MH20-22B-DR17/pEC-T18mob2thrE | 0 | 4,16 | 7,53 | 8,05 |

Folgende Figuren sind beigefügt:
- Figur 1: Karte des das Transposon Tn5531 enthaltenden Plasmids pCGL0040. Das Transposon ist als nicht schraffierter Pfeil gekennzeichnet.
- Figur 2: Karte des das thrE-Gen enthaltenden Plasmids pZ1thrE.
- Figur 3: Karte des Plasmids pEC-T18mob2.
- Figur 4: Karte des das thrE-Gen enthaltenden Plasmids pEC-T18mob2thrE.

Längenangaben sind als ca.-Angaben aufzufassen. Die verwendeten Abkürzungen und Bezeichnungen haben folgende Bedeutung:
- Amp: Ampicillinresistenzgen
- Kan: Kanamycinresistenzgen
- `amp: 3'-Teil des Ampicillinresistenzgens
- oriBR322: Replikationsregion des Plasmides pBR322
- Tet: Resistenzgen für Tetracyclin
- oriV: Plasmidkodierter Replikationsursprung von E. coli
- RP4mob: mob Region zur Mobilisierung des Plasmides
- rep: Plasmidkodierter Replikationsursprung aus C. glutamicum Plasmid pGA1
- per: Gen zur Kontrolle der Kopienzahl aus pGA1
- lacZ-alpha: lacZα-Genfragment (N-Terminus) des β-Galactosidase Gens

Die Abkürzungen für die Restriktionsenzyme haben folgende Bedeutung
- BamHI: Restriktionsendonuklease aus Bacillus amyloliquefaciens
- BglII: Restriktionsendonuklease aus Bacillus globigii
- EcoRI: Restriktionsendonuklease aus Escherichia coli
- EcoRV: Restriktionsendonuklease aus Escherichia coli
- HindIII: Restriktionsendonuklease aus Haemophilus influenzae
- KpnI: Restriktionsendonuklease aus Klebsiella pneumoniae
- PstI: Restriktionsendonuklease aus Providencia stuartii
- PvuI: Restriktionsendonuklease aus Proteus vulgaris
- SacI: Restriktionsendonuklease aus Streptomyces achromogenes
- SalI: Restriktionsendonuklease aus Streptomyces albus
- SmaI: Restriktionsendonuklease aus Serratia marcescens
- XbaI: Restriktionsendonuklease aus Xanthomonas badrii
- XhoI: Restriktionsendonuklease aus Xanthomonas holcicola

## Patentansprüche

1. In coryneformen Mikroorganismen replizierbare, rekombinante DNA mit der Herkunft Corynebacterium, die zumindest eine Nukleotidsequenz enthält, die für das thrE-Gen codiert, ausgewählt aus der Gruppe:
(i) Nukleotidsequenzen, gezeigt in SEQ-ID-No. 1,oder SEQ-ID No. 3, die für das thrE-Gen codieren, oder
(ii) mindestens einer Sequenz, die den Sequenzen (i) innerhalb des Bereichs der Degeneration des genetischen Codes entspricht, oder
(iii) Nukleotidsequenzen mit funktionsneutralen Sinnmutationen in (i).

2. Aminosäuresequenz des Proteins, abgeleitet aus den Nukleotidsequenzen gemäß den Anspruch 1, dargestellt in der SEQ-ID-No. 2 und der SEQ-ID-No. 4.

3. Threonin produzierende coryneforme Mikroorganismen, insbesondere der Gattung Corynebacterium, transformiert durch die Einführung einer oder mehrerer der replizierbaren DNA gemäß Anspruche 1.

4. Threonin produzierende Coryneforme Bakterien, insbesondere der Gattung Corynebacterium, in denen die intrazelluläre Aktivität des thrE-Enzyms, dargestellt in SEQ ID No. 2 und 4, erhöht, insbesondere die für das thrE-Gen kodierende oder diese tragende Nukleotidsequenz, dargestellt in SEQ ID No. 1 und 3, überexprimiert vorliegt.

5. Threonin produzierende Coryneforme Bakterien gemäß Anspruch 4,
**dadurch gekennzeichnet,**
**daß** die Kodierregion des thrE-Gens, dargestellt in SEQ ID No. 1 und 3,
a) funktionsneutrale Sinnmutationen enthält oder
b) mindestens eine Sequenz, die den genannten innerhalb
des Bereichs der Degeneration des genetischen Codes entspricht.

6. Threonin produzierende coryneforme Bakterien, insbesondere der Gattung Corynebacterium, transformiert durch die Einführung einer oder mehrerere der replizierbaren Nukleotidsequenzen, ausgewählt aus der Gruppe SEQ ID No. 1 Position 398 bis 1864, SEQ ID No. 1, SEQ ID No. 3 Position 280 bis 1746 und SEQ ID No. 3.

7. Corynebacterium glutamicum DM368-2 pZ1thrE, hinterlegt unter der Nummer DSM 12840.

8. Verfahren zur Herstellung von L-Threonin durch Fermentation coryneformer Bakterien,
**dadurch gekennzeichnet,**
**daß** man Bakterien einsetzt, in denen man die intrazelluläre Aktivität des thrE-Enzyms, dargestellt in SEQ ID No. 2 und 4, erhöht, insbesondere die für das thrE-Gen kodierende oder diese tragende Nukleotidsequenz, dargestellt in SEQ ID No. 1 und 3, überexprimiert.

9. Verfahren zur Herstellung von L-Threonin durch Fermentation coryneformer Bakterien, in denen man die intrazelluläre Aktivität des thrE-Enzyms, dargestellt in SEQ ID No. 4, erhöht, insbesondere die für das thrE-Gen kodierende oder diese tragende Nukleotidsequenz, enthalten in dem Plasmid pZ1thrE und hinterlegt in Corynebacterium glutamicum DSM12840, überexprimiert.

10. Verfahren gemäß Anspruch 8,
**dadurch gekennzeichnet,**
**daß** man Bakterien einsetzt, bei denen die Kodierregion des thrE-Gens, dargestellt in SEQ ID No. 1 und 3,
a) eine funktionsneutrale Sinnmutation enthält oder
b) eine Sequenz, die den genannten innerhalb des Bereichs der Degeneration des genetischen Codes entspricht.

11. Verfahren nach den Ansprüchen 8 bis 10,
**dadurch gekennzeichnet,**
**daß** man einen mit einem Plasmidvektor transformierten Stamm einsetzt und der Plasmidvektor die für das thrE-Gen kodierende Nukleotidsequenz trägt, dargestellt in SEQ ID No. 1 und 3.

12. Verfahren gemäß Anspruch 11,
**dadurch gekennzeichnet,**
**daß** man
a) mit dem Plasmidvektor pZ1thrE, hinterlegt in Corynebacterium glutamicum DSM12840, dargestellt in Figur 2, oder
b) mit dem Plasmidvektor pEC-T18mob2thrE, dargestellt in Figur 4,
transformierte Stämme einsetzt.

13. Verfahren gemäß den Ansprüchen 8 bis 12,
**dadurch gekennzeichnet,**
**daß** man gleichzeitig das für die Homoserin-Dehydrogenase kodierende hom-Gen überexprimiert.

14. Verfahren gemäß den Ansprüchen 8 bis 12,
**dadurch gekennzeichnet,**
**daß** man gleichzeitig ein für eine feed back resistente Homoserin-Dehydrogenase kodierende hom^{dr}-Allel überexprimiert.

15. Verfahren gemäß den Ansprüchen 8 bis 12,
**dadurch gekennzeichnet,**
**daß** man gleichzeitig das für die Pyruvat-Carboxylase kodierende pcy-Gen überexprimiert.

16. Verfahren gemäß den Ansprüchen 8 bis 12,
**dadurch gekennzeichnet,**
**daß** man gleichzeitig das für die Malat-Chinon:Oxidoreduktase kodierende mqo-Gen überexprimiert.

17. Verfahren gemäß den Ansprüchen 8 bis 16,
**dadurch gekennzeichnet,**
**daß** man gleichzeitig das für die Threonin-Dehydrogenase kodierende Gen ausschaltet.

18. Verfahren zur fermentativen Herstellung von L-Threonin gemäß einem oder mehreren der vorhergehenden Ansprüche, **gekennzeichnet, durch** die Schritte:
a) Fermentation der L-Threonin produzierenden Bakterien, gemäß den Ansprüchen 3 bis 7,
b) Anreicherung des L-Threonins im Medium oder in den Zellen der Bakterien, und
c) Isolieren des produzierten L-Threonins.

19. Verfahren gemäß den Ansprüchen 8 bis 18,
**dadurch gekennzeichnet,**
**daß** man Bakterien der Gattung Corynebacterium einsetzt, die bereits L-Threonin produzieren.

20. Verfahren zur Isolierung des thrE-Gens, dargestellt in SEQ ID No: 1 und 3
**dadurch gekennzeichnet,**
**daß** man als Indikatorstämme im genannten Gen defekte Mutanten, vorzugsweise coryneformer Bakterien gewinnt, die auf einem ein Threonin-haltiges Oligopeptid enthaltenden Nährmedium nicht oder nur gering wachsen und
a) das genannte Gen nach dem Anlegen einer Genbank identifiziert und isoliert, oder
b) im Fall der Transposon-Mutagenese auf das bevorzugt eine Antibiotikaresistenz enthaltende Transposon selektiert und so das genannte Gen erhält.

## Claims

1. Recombinant DNA replicable in coryneform microorganisms and derived from Corynebacterium that contains at least one nucleotide sequence that codes for the thrE gene, selected from the group:
(i) nucleotide sequences shown in SEQ-ID-No. 1 or SEQ-ID No. 3 that code for the thrE gene, or
(ii) at least one sequence that corresponds to the sequences (i) within the region of degeneracy of the genetic code, or
(iii) nucleotide sequences with functionally neutral sense mutations in (i).

2. Amino acid sequence of the protein derived from the nucleotide sequences according to claim 1, shown in SEQ-ID-No. 2 and SEQ-ID No. 4.

3. Threonine-producing coryneform microorganisms, in particular of the genus Corynebacterium, transformed by the introduction of one or more of the replicable DNA according to claim 1.

4. Threonine-producing coryneform bacteria, in particular of the genus Corynebacterium, in which the intracellular activity of the thrE enzyme shown in SEQ-ID-Nos. 2 and 4 is raised, in particular the nucleotide sequence coding for the thrE gene or carrying this, shown in SEQ-ID-Nos. 1 and 3, is present overexpressed.

5. Threonine-producing coryneform bacteria according to claim 4, **characterised in that** the coding region of the thrE gene shown in SEQ-ID-Nos. 1 and 3
a) contains functionally neutral sense mutations or
b) at least one sequence that corresponds to the aforementioned within the region of degeneracy of the genetic code.

6. Threonine-producing coryneform bacteria, in particular of the genus Corynebacterium, transformed by the introduction of one or more of the replicable nucleotide sequences selected from the group SEQ-ID-No. 1 position 398 to 1864, SEQ-ID-No. 1, SEQ-ID-No. 3 position 280 to 1746 and SEQ-ID-No. 3.

7. Corynebacterium glutamicum DM368-2 pZ1thrE deposited under No. DSM 12840.

8. Process for the production of L-threonine by fermentation of coryneform bacteria, **characterised in that** bacteria are used in which the intracellular activity of the thrE enzyme, shown in SEQ-ID-Nos. 2 and 4, is raised, in particular the nucleotide sequence coding for the thrE gene or carrying the latter, shown in SEQ-ID-Nos. 1 and 3, is overexpressed.

9. Process for the production of L-threonine by fermentation of coryneform bacteria, in which the intracellular activity of the thrE enzyme, shown in SEQ-ID-No. 4, is raised, in particular the nucleotide sequence coding for the thrE gene or carrying the latter, contained in the plasmid pZ1thrE and deposited in Corynebacterium glutamicum DSM 12840, is overexpressed.

10. Process according to claim 8, **characterised in that** bacteria are used in which the coding region of the thrE gene, shown in SEQ-ID-Nos. 1 and 3
a) contains a functionally neutral sense mutation or
b) a sequence that corresponds to the aforementioned within the region of degeneracy of the genetic code.

11. Process according to one of claims 8 to 10, **characterised in that** a strain transformed with a plasmid vector is used and the plasmid vector carries the nucleotide sequence coding for the thrE gene, shown in SEQ-ID-Nos. 1 and 3.

12. Process according to claim 11, **characterised in that** strains are used that have been transformed
a) with the plasmid vector pZ1thrE, deposited in Corynebacterium glutamicum DSM 12840, shown in Fig. 2, or
b) with the plasmid vector pEC-T18mob2thrE shown in Fig. 4.

13. Process according to claims 8 to 12, **characterised in that** the hom gene coding for homoserine dehydrogenase is simultaneously overexpressed.

14. Process according to claims 8 to 12, **characterised in that** a hom^{dr} allele coding for a feedback-resistant homoserine dehydrogenase is simultaneously overexpressed.

15. Process according to claims 8 to 12, **characterised in that** the pyc gene coding for pyruvate carboxylase is simultaneously overexpressed.

16. Process according to claims 8 to 12, **characterised in that** the mqo gene coding for malate-quinone oxidoreductase is simultaneously overexpressed.

17. Process according to claims 8 to 16, **characterised in that** the gene coding for threonine dehydrogenase is simultaneously switched off.

18. Process for the fermentative production of L-threonine according to one or more of the preceding claims, **characterised by** the following steps:
a) fermentation of the bacteria producing L-threonine according to claims 3 to 7,
b) enrichment of the L-threonine in the medium or in the bacterial cells, and
c) isolation of the produced L-threonine.

19. Process according to claims 8 to 18, **characterised in that** bacteria of the genus Corynebacterium that already produce L-threonine are employed.

20. Process for the isolation of the thrE gene, shown in SEQ-ID-Nos. 1 and 3, **characterised in that** as indicator strains mutants defective in the aforementioned gene, preferably coryneform bacteria, are obtained that do not grow or grow only slightly on a nutrient medium containing a threonine-containing oligopeptide, and
a) 'the aforementioned gene is identified and isolated after the establishment of a gene bank, or
b) in the case of transposon mutagenesis is selected for the transposon preferably exhibiting a resistance to antibiotics, and the aforementioned gene is thereby obtained.

## Revendications

1. ADN recombinant d'origine Corynebacterium capable de se répliquer dans des microorganismes corynéformes, qui contient au moins une séquence de nucléotides, qui sert de code au gène thrE, sélectionné parmi le groupe suivant :
(i) séquences de nucléotides, représentées dans les SEQ-ID-n° 1 ou SEG-ID-n° 3, qui servent de code au gène thrE, ou
(ii) d'au moins une séquence qui correspond aux séquences (i) relevant du domaine de la dégénérescence du code génétique, ou
(iii) des séquences de nucléotides avec des mutations utiles de (i) neutres sur le plan des fonctions.

2. Séquence d'acides aminés de la protéine, dérivée des séquences de nucléotides selon la revendication 1, représentée dans la SEQ-ID-n° 2 et la SEQ-ID-n° 4.

3. Microorganismes corynéformes produisant la thréonine, en particulier du genre Corynebacterium, transformés par l'introduction d'un ou de plusieurs ADN capables de se répliquer, selon la revendication 1.

4. Bactéries corynéformes produisant la thréonine en particulier du genre Corynebacterium, dans lesquelles on augmente l'activité intracellulaire de l'enzyme thrE, représentée dans les SEQ-ID-n° 2 et 4, et on surexprime en particulier la séquence de nucléotides servant de code au gène thrE ou portant celles-ci, représentée dans les SEQ-ID-n° 1 et 3.

5. Bactéries corynéformes produisant la thréonine selon la revendication 4,
**caractérisées en ce que** la région de codage du gène thrE, représentée dans les SEQ-ID-n° 1 et 3,
a) contient des mutations utiles neutres sur le plan des fonctions, ou
b) au moins une séquence qui correspond aux citées, relevant du domaine de la dégénérescence du code génétique.

6. Bactéries corynéformes produisant la thréonine, en particulier du genre Corynebacterium, transformées par l'introduction d'une ou de plusieurs séquences de nucléotides capables de se répliquer, sélectionnées parmi le groupe SEQ-ID-n° 1 positions 398 à 1864, SEQ-ID-n° 1, SEQ-ID-n° 3 positions 280 à 1746 et SEQ-ID-n° 3.

7. Corynebaoterium glutamicum DM368-2 pZ1thrE, déposé sous le numéro DSM 12840.

8. Procédé de préparation de la L-thréonine par fermentation de bactéries corynéformes,
**caractérisé en ce qu'**on utilise des bactéries dans lesquelles on augmente l'activité intracellulaire de l'enzyme thrE, représentée dans les SEQ-ID-n° 2 et 4, on surexprime en particulier la séquence de nucléotides servant de code au gène thrE ou portant celles-ci, représentée dans les SEQ-ID-n° 1 et 3.

9. Procédé de préparation de L-thréonine par fermentation de bactéries corynéformes, dans lesquelles on augmente l'activité intracellulaire de l'enzyme thrE, représentée à la SEQ-ID-n° 4, on surexprime en particulier la séquence de nucléotides servant de code au gène thrE ou portant celles-ci, contenu dans le plasmide pZ1thrE et déposé dans le Corynebacterium glutamicum DSM12840.

10. Procédé selon la revendication 8, **caractérisé en ce qu'**on utilise des bactéries, dans lesquelles la région de codage du gène thrE, représenté dans les SEQ-ID-n° 1 et 3,
a) contient une mutation utile neutre sur le plan des fonctions ou
b) une séquence qui correspond à la citée relevant du domaine de la dégénérescence du code génétique.

11. Procédé selon les revendications 8 à 10,
**caractérisé en ce qu'**on utilise une souche transformée par un vecteur plasmide et que le vecteur plasmide porte la séquence de nucléotides servant de code au gène thrE, représenté aux SEQ-ID-n° 1 et 3.

12. Procédé selon la revendication 11,
**caractérisé en ce qu'**on utilise des souches transformées
a) par le vecteur plasmide pZ1thrE, déposé dans le Corynebacterium gutamicum DSM12840, représenté à la Fig. 2, ou
b) par le vecteur plasmide pEC-T18mob2thrE, représenté à la Fig. 4.

13. Procédé selon les revendications 8 à 12,
**caractérisé en ce qu'**on surexprime en même temps le gène hom servant de code à l'homo-sérine-déshydrogénase.

14. Procédé selon les revendications 8 à 12,
**caractérisé en ce qu'**on surexprime en même temps un allèle hom^{dr} servant de code à une homo-sérine-déshydrogénase résistant au feed-back.

15. Procédé selon les revendications 8 à 12,
**caractérisé en ce qu'**on surexprime en même temps le gène pcy servant de code à la pyruvate-carboxylase.

16. Procédé selon les revendications 8 à 12,
**caractérisé en ce qu'**on surexprime en même temps le gène mqo servant de code à la malate-quinone:oxydoréductase.

17. Procédé selon les revendications 8 à 16,
**caractérisé en ce qu'**on élimine en même temps le gène servant de code à la thréonine-déshydrogénase.

18. Procédé de préparation par fermentation de L-thréonine selon l'une ou plusieurs des revendications précédentes, **caractérisé par** les étapes suivantes :
a) fermentation des bactéries produisant la L-thréonine, selon les revendications 3 à 7,
b) enrichissement de la L-thréonine en milieu ou dans les cellules des bactéries, et
c) isolement de la L-thréonine produite.

19. Procédé selon les revendications 8 à 18,
**caractérisé en ce qu'**on utilise des bactéries du genre Corynebacterium qui produisent déjà la L-thréonine.

20. Procédé d'isolation du gène thrE, représenté dans les SEQ-ID-n° 1 et 3
caractérisé en cequ'on obtient comme souches indicatrices dans le gène cité des mutants défectueux, de préférence des bactéries corynéformes qui ne croissent pas ou seulement peu sur un milieu de culture contenant un oligopeptide contenant la thréonine, et
a) on identifie et le gène cité sur la base d'une banque génétique et on l'isole, ou
b) on sélectionne, dans le cas de mutagenèse par transposon, un transposon contenant de préférence une résistance aux antibiotiques, et ainsi on obtient le gêne cité.
